# EUROPEAN PATENT SPECIFICATION

(11) **EP 1 062 310 B1**
(45) Date of publication and mention of the grant of the patent: **09.08.2006**
(21) Application number: 99911265.9
(22) Date of filing: 10.03.1999
(51) Int. Cl.: C11D 1/66

(54) **IMPROVED ALKANOLAMIDES**
VERBESSERTE ALKANOLAMIDE
ALCANOLAMIDES AMELIORES

(30) Priority: 11.03.1998 US 38736
(43) Date of publication of application: 27.12.2000
(73) Proprietor: MONA INDUSTRIES, INC., Paterson New Jersey 07544 (US)
(72) Inventor: PERELLA, James, E., Mahwah, NJ 07430 (US); KOMOR, Joseph, A., Ramsey, NJ 07446 (US); FOST, Dennis, L., Ridgewood, NJ 07450 (US); KATSTRA, Richard, D., Warwick, NY 10990 (US)
(74) Representative: Humphries, Martyn
(86) International application number: PCT/US1999/005177
(87) International publication number: WO 1999/046356

(56) References cited:
- DE-A1- 2 643 804
- JP-A- 4 136 289
- JP-A- 8 337 560
- JP-A- 9 067 325
- JP-A- 61 064 322
- DATABASE CA [Online] CHEMICAL ABSTRACTS SERVICE, COLUMBUS, OHIO, US; retrieved from STN Database accession no. 96:11503/DN, HCAPLUS XP002212792 & JP 56 082897 A (SANYO CHEMICAL IND.) 6 July 1981 (1981-07-06)
- DATABASE CA [Online] CHEMICAL ABSTRACTS SERVICE, COLUMBUS, OHIO, US; retrieved from STN Database accession no. 129:344751/DN, HCAPLUS XP002212793 -& JP 10 279996 A (KAO CORP.) 20 October 1998 (1998-10-20)

## Description

### Field of the Invention

The present invention relates to improved alkanolamide surfactants and, more particularly, to modified monoalkanolamides which are liquid at ambient temperatures and to the method of making the same.

### Background of the Invention

Nonionic surfactants are well known and have achieved fast growing commercial importance. They encompass a broad range of compounds having a diverse range of structures and applications. One type of nonionic surfactants are alkanolamides that are condensates, for example, of fatty acids with alkanolamines such as monoethanolamine (MEA), diethanolamine (DEA) and monoisopropanolamine (MIPA), have been used in a variety of cosmetic, personal care, household and industrial formulations.

Alkanolamides are widely used in generally liquid systems such as liquid detergents and personal care products as foam stabilizers, viscosity builders, solubilizers and the like, in metal working formulations as lubricants, viscosity control agents, corrosion inhibitors and in a variety of other applications. Alkanolamides utilized as components in such systems are ethanolamides and/or isopropanolamides such as monoethanolamides, diethanolamides and isopropanolamides in which the fatty acid acyl radical typically contains from 8 to 18 carbon atoms. Such dialkanolamides are typically liquid, while monoalkanolamides are solids having melting points of 40°C to about 90°C. Heretofore, especially satisfactory alkanolamides have been diethanolamides such as those derived from coconut oil mixed fatty acids or special fractions containing, for instance, predominately C₁₂ to C₁₄ fatty acids. These alkanolamides are generally liquid in form which greatly simplifies their use.

Up to now, monoalkanolamides have not been available in liquid form which has limited their use in many applications. In recent years, because of regulatory concerns and restrictions, formulation trends have been shifting toward greater usage of monoalkanolamides; such products being solids, are more difficult to handle and are inconvenient to use in large scale production processes. Accordingly, it would be highly advantageous to be able to combine the performance characteristics of monoethanolamides with products having liquid physical characteristics at ambient conditions or lower.

Another well known basic group of nonionic surfactants are the polyoxylated derivatives, primarily represented by polyethoxylated and polypropoxylated compounds which are widely used as emulsifiers and detergents but do not provide the advantages of alkanolamides in connection with, for example, foam stabilization and viscosity building. Attempts have been made in the past to use ethylene oxide as an adduction agent for alkanolamides to modify the properties of the alkanolamides in a favorable manner including possibly reducing the congealing temperature of the monoethanolamides. While ethylene oxide adducts of alkanolamides were found to effect compositions with some modified properties, the amount of ethylene oxide needed to achieve physical property changes in the alkanolamides resulted in products which more closely resembled those exhibited by polyoxyethylene compounds, and the derivatives did not retain many of the characteristics of alkanolamides which were most desirable.

**JP8337560-A** is directed to mixtures of polyoxypropylene fatty acid alkanolamide compounds prepared by reacting a fatty acid alkyl ester with an alkanolamide, followed by optional ethoxylation and then propoxylation.

**WO-9208690-A** discloses a method for producing ethanolamide ethoxylate in the presence of a tertiary amine lacking protons that react with alkylene oxide.

The invention further provides a method for preparing a modified monoethanolamide surfactant composition having a congealing temperature of 20°C or lower which comprises
(a) providing a monoethanolamide composition, prepared from triglyceride, represented by the formula (2) 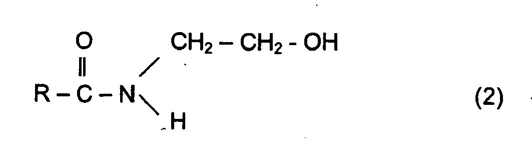 wherein
   R is optionally substituted or unsubsitututed, branched or straight chain, saturated or unsaturated, hydrocarbon radical of 3-21 carbon atoms; and
b) reacting 1 mole of said monoethanolamide composition in the presence of a suitable catalyst with 1-6 moles of propylene oxide, butylene oxide or mixtures of the same, to form a modified monoethanolamide surfactant composition that is substantially liquid at 25°C or lower.

Accordingly the present invention provides a modified monoethanolamide surfactant composition derived from monoethanolamide prepared from triglyceride, having a congealing temperature of 20°C or lower of the formula (1) 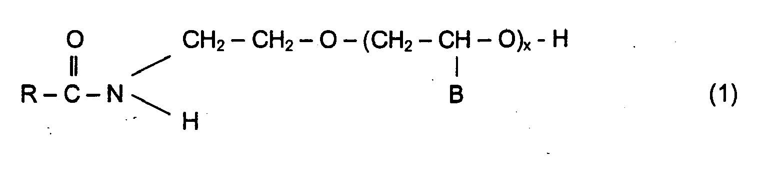 wherein
R is optionally substituted or unsubstituted, branched or straight chain, saturated or unsaturated, hydrocarbon radical of 3-21 carbon atoms,
B is CH₃ or -CH₂ - CH₃, and
x is from 1 to 6;
said modified monoethanolamide composition being substantially liquid at ambient temperature or lower, and being suitable to exhibit foam stabilization and viscosity building properties.

The invention also provides the use of a monoethanolamide composition prepared from glyceride esters, to form a modified monoethanolamide surfactant composition of the formula (1) 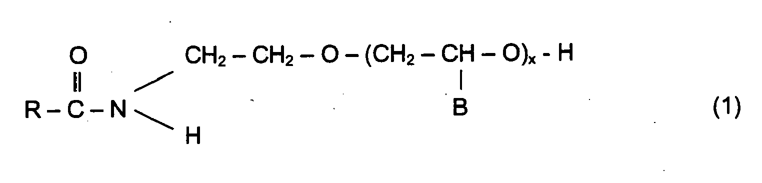 wherein
R is optionally substituted or unsubstituted, branched or straight chain, saturated or unsaturated or unsaturated hydrocarbon radical of 3-21 carbon atoms,
B is CH₃ or -CH₂ - CH₃, and
x is from 1 to 6;
having a congealing temperature of 20°C or lower, and being substantially liquid at 25°C or lower."

### Summary of the Invention

It is accordingly an object of the present invention to provide a process for preparing modified monoethanolamide surfactant compositions having a congealing temperature less than 25°C, that preferably are liquids at ambient temperature or lower (e.g. 25°C or lower), and that exhibit the foam stabilizing, viscosity building and other desirable surfactant characteristics of unmodified dialkanolamide and monoalkanolamide surfactants.

It is another object of the present invention to provide a novel process for preparing an improved monoethanolamide surfactant composition by reacting a monoethanolamide surfactant composition having a congealing temperature of about 40°C or higher with an amount of propylene or butylene oxide sufficient to prepare a modified monoethanolamide surfactant composition having a congealing temperature of 20°C or lower that substantially exhibits the surfactant characteristics of dialkanolamide surfactant compositions and the monoethanolamide reactant.

It is a further object of the present invention to provide an improved modified monoethanolamide surfactant composition having a congealing temperature lower than 20°C, that preferably is liquid at ambient temperature (25°C) or lower and that exhibits foam stabilizing, viscosity building and the like characteristics of dialkanolamide and monoalkanolamide surfactants.

It is still a further object to provide cosmetic and other personal care preparations containing modified monoethanolamide surfactants having congealing temperatures of 20°C or lower.

It is yet another object of the present invention to provide metal working and other compositions for a variety of household and industrial applications containing modified monoethanolamide surfactants having congealing temperatures of 20°C or lower.

These and other objects will become apparent from the description to follow.

The modified monoethanolamide compositions of the present invention which are substantially liquids at ambient temperature or lower, preferably, have a congealing temperature of 20°C or, most preferably lower, surprisingly and unexpectedly generally exhibit most, if not all, of the surfactant characteristics of dialkanolamide surfactant compositions such as the foam stabilization and viscosity building properties, as well as retaining substantially all the favorable properties of the monoethanolamides from which the compositions are prepared.

In yet another aspect of the present invention, there are provided cosmetic and personal care compositions which comprise at least 0.1% by weight of a modified monoethanolamide surfactant composition prepared from triglycerides which is substantially a liquid at ambient temperature or lower that may be represented by the formula 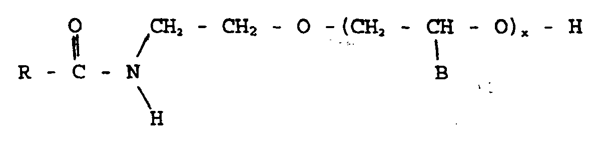 Wherein:
- R: is optionally substituted or unsubstituted, branched or straight chain, saturated or unsaturated hydrocarbon radical of 3-21 carbon atoms;
- B: is CH₃ or -CH₂ - CH₃; and
- x: is from 1 to 6.

In a still further aspect of the present invention there are provided metal working compositions and household cleaning products which are preferably in liquid form comprising at least 0.1% by weight of a modified monoethanolamide surfactant composition prepared from triglycerides which is substantially a liquid at ambient temperature or lower that may be represented by the formula 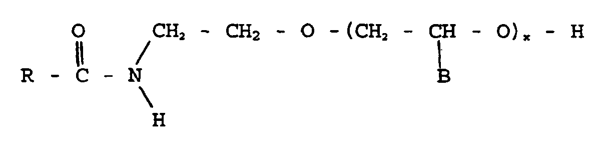 Wherein:
- R: is optionally substituted or unsubstituted, branched or straight chain, saturated or unsaturated hydrocarbon radical of 3-21 carbon atoms;
- B: is CH₃ or -CH₂ - CH₃; and
- x: is from 1 to 6.

### Description of the Preferred Embodiments

In accordance with the present invention, materials and methods are provided which enhance the properties of a well known and widely used class of nonionic surfactant compositions making an easily handleable group thereof readily and economically available for use in a variety of cosmetic, personal care, household and industrial applications.

The method of this invention applies to the treatment of a monoethanolamide surfactant composition generally having a congealing temperature of 40°C or higher by reacting the normally solid monoethanolamide composition in the presence of a suitable catalyst, preferably potassium hydroxide or sodium alcoholate, with an amount of butylene oxide or preferably propylene oxide only sufficient to prepare a monoethanolamide derivative composition which is substantially liquid at ambient temperatures ( 25°C) and preferably has a congealing temperature of 20°C or lower. The monoethanolamide derivative compositions which are prepared in accordance with the practice of the present invention exhibit surfactant properties such as foam stabilization and viscosity building as well as other desirable characteristics which are similar to those exhibited by dialkanolamides and are substantially the same as those provided by the unmodified monoethanolamide compositions. Moreover, the monoethanolamide derivative compositions of the present invention which are substantially liquid at ambient temperatures or lower, are not known to have any undesirable toxicological or environmental concerns.

In general, the method of the present invention can be accomplished by treating a monoethanolamide composition which has the formula 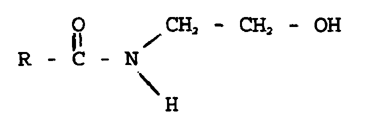 wherein:
- R: is optionally substituted or unsubstituted, branched or straight chain, saturated or unsaturated hydrocarbon radical of 3 - 21, preferably 8- 18 carbon atoms;
with an amount of propylene oxide, butylene oxide or mixtures of the same in the presence of a suitable catalyst, such as potassium hydroxide, sodium alcoholate and the like, that is only sufficient to form a monoethanolamide derivative composition which is liquid at ambient temperatures or lower and, preferably, substantially retains the surfactant characteristics of the unmodified monoethanolamide composition.

The reaction of the monoethanolamide composition with propylene oxide, butylene oxide or mixtures of the same for adding one or more moles of alkylene oxide onto the alkanolamide in accordance with the invention can be carried out using any well known method. The degree of alkoxylation of the monoethanolamide composition being treated is important but may be varied depending upon the molecular weight of the monoethanolamide composition and the degree of unsaturation in the fatty alkyl amide moiety as expressed by the iodine value thereof, by adding from 1 to 6 moles, preferably from 1 to 4 moles, of propylene oxide, butylene oxide or mixtures thereof, per mole of monoethanolamide. However, the number of moles of alkylene oxide used, as indicated, should be only the amount sufficient to produce a monoethanolamide derivative composition which is substantially liquid at ambient temperatures, and, preferably, has a congealing temperature of 20°C or, more preferably, lower.

The typically solid monoethanolamide compositions suitable for use in the preparation of modified monoethanolamide compositions of the present invention having a congealing temperature of 20°C or preferably lower in accordance with the practice of the present invention are well known and include those derived from triglycerides. Examples of suitable triglycerides from which the monoethanolamide compositions may be prepared include glyceride esters as are found in coconut oil, palm oil, sunflower oil, soybean oil, rapeseed oil, castor oil, fish oil, tallow fat, milk fat, lard and other natural sources or may be of synthetic origin.

The modified monoethanolamide surfactant compositions of the present invention which are prepared in accordance with the practice of the present invention may be represented by the formula 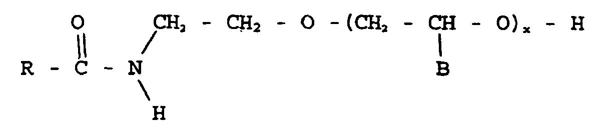 Wherein:
- R: is optionally substituted or unsubstituted, branched or straight chain, saturated or unsaturated hydrocarbon radical of 3-21, preferably, 8-18 carbon atoms;
- B: is -CH₃ or -CH₂ - CH₃; and

As indicated, the monoethanolamide compositions which are typically high temperature melting waxy solids that are converted into the monoethanolamide derivatives of the present invention may be prepared in any suitable manner and numerous processes for their productions are well known. The modified monoethanolamide surfactant compositions of the present invention, which are liquids at ambient temperatures and, preferably, have a congealing temperature of 20°C or lower, retain the generally useful and desirable surfactant and other properties of the monoethanolamide compositions from which they are prepared as well as those exhibited by diethanolamide compositions that are well known and widely used.

The degree of alkoxylation, that is, the number of groupings "x" of the above formula, may be varied but only within narrow limits. Monoethanolamide derivative compositions of the invention which are adducts of only a sufficient amount of butylene oxide or, preferably, propylene oxide per mole of monoethanolamide, from 1-6 moles, preferably from 1-4 moles, of alkylene oxide, surprisingly and unexpectedly are liquids at ambient temperatures and preferably have a congealing temperature of 20°C or lower while advantageously retaining the desirable surfactant characteristics of the unmodified monoethanolamide compositions as well as displaying the surfactant properties of diethanolamides. Depending on the molecular weight and degree of unsaturation of the fatty alkyl amide moiety of the monoethanolamide composition as expressed by the iodine value (I.V.) thereof, the monoethanolamide derivative compositions of the present invention will contain an amount of propylene oxide to monoethanolamide derivative composition in the range from at least 22% by weight to 57% by weight, or an amount of butylene oxide to monoethanolamide derivative composition in the range from 40% by weight to 60% by weight, although in the case of monoethanolamide compositions containing saturated high molecular weight amide moieties (iodine values of 0), the amount of propylene oxide and/or butylene oxide to monoethanolamide derivative of about 60% by weight or higher may prove to be desirable.

The novel modified monoethanolamide compositions of the invention display many of the well known properties of diethanolamides such as foam stabilization and viscosity building as well as other desirable properties such as emulsification of oil based materials, solubilization of fragrances and hair colorants and dyes, wetting of natural and synthetic fibers, compatability with anionic surfactants and detergents and the like while exhibiting congealing temperatures which are substantially lower than those of the unmodified monoethanolamide compositions, thus making them easy to formulate into a wide range of cosmetic, personal care, household and industrial systems. Surprisingly and unexpectedly it has been found that while alkoxylation of monoethanolamides with controlled, small amounts of propylene oxide or butylene oxide in accordance with the practice of the invention provides monoethanolamide derivatives which are liquid at ambient temperatures or lower and retain all or most of the desirable surfactant characteristics of the unmodified monoethanolamides and of diethanolamides, reactions which involve treating typically solid monoethanolamide compositions with 5 or more moles of ethylene oxide in the presence of a suitable catalyst are useful in forming derivatives thereof which are flowable solids or gels at ambient temperatures (20-25°C) but the monoethanolamide derivatives thus formed, significantly lack many of the desirable alkanolamide surfactant properties such as foam stabilization and viscosity building.

It is therefore a further aspect of the invention to use the novel compositions of the invention, for example, in cosmetic preparations and the like, especially in preparations for hair care and skin treatment. In this connection, personal care formulations can be shampoos, hair colorants, hair conditioners, bath products and skin treatment cremes and lotions. Other formulations where the properties of the novel compositions of the invention can be utilized include make-up cremes, sunscreens, lipstick, pressed powders, skin-toners, antiperspirants and the like. Shampoos to which the novel modified monoethanolamide compositions of the invention have been added in amounts of from 0.1 to 10 percent by weight or greater and which contain up to about 30 weight percent of substances with detergent activity, in addition to water and possibly other ingredients, result in compositions having desired foam stability and viscosity building as well as many other desirable characteristics.

Conventional additives such as perfumes, preservatives, complexing agents, opacifiers, luster developing agents and the like may be added to any of the above mentioned personal care products.

The novel modified monoethanolamide compositions may also be added to a wide variety of home care and industrial formulations wherein their usefulness as detergents, metal working and lubricating agents, emulsifiers, anticorrosion agents for metal products and various other liquid and/or water based personal care, home care and industrial compositions wherein diethanolamide and unmodified monoethanolamide compositions have been found useful. The preparation of specific compositions of the invention is illustrated by the following examples which are provided herein for purposes of illustration only and are not intended to limit the scope thereof.

### Example 1

A three-necked flask fitted with an agitator, thermometer, dry-ice condenser, heating mantle and addition funnel is charged with 125 grams of a solid commercially available coconut monoethanolamide having the tradename MONAMID CMA, which is prepared from coconut oil and monoethanolamine. The solid monoethanolamide (congealing temperature 63°C; Iodine Value (I.V.)- 9) is melted at 70 - 80°C, 0.9 grams of 85% potassium hydroxide catalyst are added and the mixture is stirred until homogeneous. The temperature of the reaction mixture is raised to 140-150°C and 58 grams of propylene oxide is added dropwise over a period of 1.5 hours. The reaction mixture is stirred at 140-150°C for an additional hour after which it is cooled to 25°C. The propoxylated monoethanolamide product with a propylene oxide content of 31.5% by weight is a clear amber liquid having a congealing temperature of -1°C.

### Example 2 (outside the claimed range)

Using the reaction vessel of example 1, 125 grams of a solid commercially available coconut monoethanolamide having the tradename MONAMID CMA-A, which is prepared from coconut fatty acid and monoethanolamine, is reacted with 58 grams of propylene oxide using the procedure of example 1, A clear amber liquid product is prepared having a congealing temperature of 0°C.

### Example 3

A) Using the reaction vessel and procedure of example 1, a propoxylated monoethanolamide is prepared from 125 grams of the coconut monoethanolamide material of example 1 except that only 29 grams of propylene oxide is added to the heated monoethanolamide reactant. After completion of the reaction, the product with a propylene oxide content of 18.7% by weight is cooled to 25°C and a nonclear pasty liquid results.
B) Using the reaction vessel and procedure of example 1, a propoxylated monoethanolamide is prepared from 125 grams of the coconut monoethanolamide material of example 1 except that 174.3 grams of propylene oxide is added to the heated monoethanolamide reaction mixture. After completion of the reaction and cooling, a reaction product with a propylene oxide content of 58.2% by weight which is a clear amber liquid is obtained.
C) Using the reaction vessel and procedure of example 1, a propoxylated monoethanolamide is prepared from 125 grams of the coconut monoethanolamide material of example 1 except that 232.4 grams of propylene oxide is added to the heated monoethanolamide reaction mixture. After completion of the reaction and cooling, a clear dark amber liquid reaction product is obtained having a propylene oxide content of 65.0% by weight.

### Example 4

A) Using the reaction vessel of example 1, 192.6 grams of a soya monoethanolamide (tan solid - congealing temperature 45°C; Iodine Value - 130) prepared from soybean oil and monoethanolamine is charged into the reactor and heated at 70-80°C together with 1.5 grams of flake 85% potassium hydroxide. The mixture is agitated until homogeneous and the temperature is then raised to 140-150°C. While maintaining the temperature with agitation, 104.6 grams of propylene oxide is added dropwise over a period of 2.5 hours. The reaction mixture is stirred for an additional hour at 140-150°C and then cooled to 25°C. After cooling, the reaction product with a propylene oxide content of 35.0% is a clear amber liquid having a congealing temperature of -1°C.
B) The reaction of A) above is run except that only 69.76 grams of propylene oxide is added dropwise to the molten soya monoethanolamide reactant. The resultant product having a propylene oxide content of 26.4% is a paste at 25°C.

### Example 5

Using the reaction vessel of example 1, 230.1 grams of a caprylic/capric monoethanolamide (I.V. - 0) prepared from a C₈/C₁₀ triglyceride and monoethanolamine is charged into the reactor with 1.5 grams of 85% flake potassium hydroxide and heated at 70-80°C with agitation until a homogeneous mixture is formed. The temperature of the reaction mixture is then raised to 140-150°C and 68.4 grams of propylene oxide is added dropwise over a period of 1.5 hours. The reaction mixture is stirred for another hour at a temperature of 140-150°C. The reaction product with a propylene oxide of 22.8% by weight is a clear amber liquid at 25°C.

### Example 6

A) Using the reaction vessel of example 1, 125 grams of the solid coco monoethanolamide of example 1 is charged into the reaction vessel and melted at 70-80°C at which time 0.9 grams of 85% potassium hydroxide is added and the mixture is stirred until homogeneous. The temperature of the reaction mixture is raised to 140-150°C and 108 grams of butylene oxide are added dropwise to the reaction mixture with stirring over a period of 1.5 hours. The reaction mixture is maintained at 140-150°C with stirring for an additional hour, after which it is cooled to 25°C. The reaction product with a butylene oxide content of 46.2% is a clear amber liquid.
B) The reaction of A) above is run except that only 72 grams of butylene oxide are added dropwise to the coco alkanolamide reaction mixture over a period of 2.5 hours. After cooling, the reaction product having a butylene content of 36.4% is a solid.
C) The reaction of A) above is run except that 180 grams of butylene oxide are added dropwise to the coco alkanolamide reaction mixture over a period 2.5 hours. After cooling, the reaction product with a butylene oxide content of 59% is a clear amber liquid.

### Example 7

The propoxylated monoethanolamide derivative compositions of examples 1, 4A and 5 are used in this example. Foam stabilization properties of various modified monoethanolamide compositions are evaluated and the properties effected by the monoethanolamide derivative compositions (all having congealing temperatures less than 0°C) of the present invention are compared with those obtained with a commercial diethanolamide composition, an unmodified monoethanolamide (solid with a congealing temperature of 63°C) and a commercial monoethanolamide reacted with 5 moles of ethylene oxide (congealing temperature 25°C). The results of the tests are reported below in Table 1.

**Table 1**

| Ross Miles Foam Test (mm of Foam) | | | |
|---|---|---|---|
| Sample Tested | At 0 Minutes | After 1 Minute | After 5 Minutes |
| Example 1 Comp. | 230 | 201 | 197 |
| Unmodified Coconut monoethanolamide MONAMID CMA | 231 | 206 | 201 |
| Example 4A Comp. | 236 | 212 | 206 |
| Example 5 Comp. | 233 | 208 | 203 |
| Coconut Diethanolamide | 234 | 204 | 201 |
| Coconut Monoethanolamide + 5 moles of Ethylene Oxide | 135 | 119 | 15 |

The test is run according to ASTM Method D1173-53 with 19% of Sodium Lauryl Sulfate + 1% test amide. 0.1% total active in 0 ppm water hardness temperature = 25°C.

As can be seen, the monoethanolamide derivative compositions with low levels of propoxylation, which are liquids, exhibit excellent foam stabilization properties when blended with an anionic surfactant, such as sodium lauryl sulfate, substantially the same as the foam stablization characteristics of coconut diethanolamide and unmodified coconut monoethanolamide. In contrast thereto, an ethylene oxide adduct of the coconut monoethanolamide is not liquid and does not stabilize foam.

### Example 8

The propoxylated monoethanolamide derivative compositions of examples 1, 4A and 5 are used in this example. Viscosity building properties of various monoethanolamide derivative compositions of the invention are evaluated and compared with the properties effected by a commercial diethanolamide composition (congealing temperature -4°C), an unmodified monoethanolamide (solid-congealing temperature 63°C) and a commercial monoethanolamide adducted with 5 moles of ethylene oxide (congealing temperature 25°C). The results are reported below in Table 2.

**Table 2**

| Viscosity Comparisons of Sodium Lauryl Sulfate Blends | |
|---|---|
| Sample Tested | Viscosity (cPs) 1% Added NaCl |
| Example 1 Comp. | 283 |
| Unmodified Coconut monoethanolamide MONAMID CMA | 625 |
| Example 4A Comp. | 120 |
| Example 5 Comp. | 130 |
| Coconut Diethanolamide | 173 |
| Coconut Monoethanolamide + 5 moles of Ethylene Oxide | 45 |

Test Formulation: 19% of Sodium Lauryl Sulfate active + 1% test sample.

While the propoxylated monoethanolamide compositions (Examples 1, 4A and 5 Compositions) exhibit somewhat reduced viscosity building characteristics compared to an unmodified coconut monoethanolamide composition, they are approximately equivalent in viscosity building to a coconut diethanolamide composition and are clearly superior to the ethylene oxide adduct of a coconut monoethanolamide.

### Example 9

The alkoxylated monoethanolamide derivative compositions of examples 1, 2 (outside the claimed range), 3B) and 3C) are used in this example. Prototype hair shampoo formulations are prepared using 30% active sodium lauryl sulfate and 3% active monoethanolamide derivative compositions. A shampoo sample is prepared for comparison purposes from a solid commercially available fatty acid derived monoethanolamide having the Trade Name MONAMID CMA-A. The various shampoo samples are evaluated for Ross-Miles Foam and Viscosity and the results are reported in Table 3, below.

**Table 3**

| Sample Tested | | | | | |
|---|---|---|---|---|---|
| | CMA-A | Comp 1 | Comp 2 | Comp 3B | Comp 3C |
| Ross-Miles Foam | | | | | |
| (mm) initial | 185 | 205 | 208 | 180 | 177 |
| 1 min. | 170 | 179 | 180 | 155 | 152 |
| 5 min. | 165 | 172 | 171 | 147 | 142 |

| Viscosity (cP) | | | | | |
|---|---|---|---|---|---|
| No Salt | 25 | 16 | 16 | 4 | 6 |
| 1% NaCl | 6,200 | 440 | 2,375 | 7.5 | 5 |

As can be seen, the monoethanolamide derivative compositions with low levels of propoxylation (example 1 and 2 compositions), which are liquids, exhibit excellent foam stabilization properties when blended with an anionic surfactant, such as sodium lauryl sulfate, substantially the same as the foam stablization characteristics of unmodified coconut monoethanolamide which is not a liquid. In contrast thereto, the coconut monoethanolamide derivative compositions with high levels of propylene oxide, example 3B) and 3C) compositions, do not stabilize foam as effectively. Moreover, the propoxylated monoethanolamide compositions with low levels of propylene oxide (Example 1 and 2 Compositions) which are liquids, exhibit significant viscosity building characteristics, whereas the propoxylated monoethanolamide compositions with high level of propylene oxide, example 3B) and 3C) compositions, exhibit poor viscosity building characteristics.

### Example 10

The alkoxylated monoethanolamide derivative compositions of examples 1 and 2 outside the claimed range are used in this example. Cotton wetting by the monoethanolamide derivative compositions of the invention are evaluated and compared to the wetting characteristic of the unmodified monoethanolamide composition of example 1. The results are reported in Table 4, below.

As can be seen, the wetting characteristics of the liquid modified monoethanolamide compositions of the invention, which are readily soluble and/or dispersible in water, are clearly superior to the wetting characteristics of a solid unmodified monoethanolamide composition.

**Table 4**

| Draves Cotton Skein Wetting Test | | |
|---|---|---|
| Concentration: 0.1% active in deionized water* | | |
| Temperature: 25°C | | |
| Method: AATCC 17-1994 | | |

| Cotton Skein Wetting Time | | |
|---|---|---|
| Unmodified Coconut Monoethanolamide Triglyceride-Derived | Composition of Example 1 | Composition of Example 2 |
| Test terminated with no wetting after 35 minutes | Wets at 39.0 seconds | Wets at 39.5 seconds |

| | | |
|---|---|---|
| *Note: The propoxylated alkanolamide compositions of examples 1 and 2 are noted to be more easily dispersible and soluble than the unmodified alkanolamide compostion. | | |

It will be evident from the above that there are other embodiments and methods, which while not expressly described above, are clearly within the scope of the invention. The description above is therefore intended to be exemplary only and the scope of this invention is to be limited solely by the appended claims.

## Claims

1. A modified monoethanolamide surfactant composition derived from monoethanolamide prepared from triglyceride, having a congealing temperature of 20°C or lower of the formula (1) 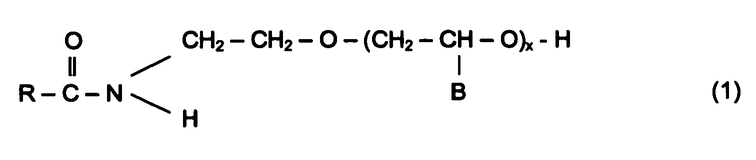 wherein
R is optionally substituted or unsubstituted, branched or straight chain, saturated or unsaturated, hydrocarbon radical of 3-21 carbon atoms,
B is CH₃ or -CH₂ - CH₃, and
x is from 1 to 6;
said modified monoethanolamide composition being substantially liquid at 25°C or lower, and being suitable to exhibit foam stabilization and viscosity building properties.

2. A surfactant composition according to claim 1 wherein x is from 1 to 4.

3. A surfactant composition according to either one of claims 1 and 2 wherein B is a CH₃ group.

4. A surfactant composition according to any one of the preceding claims wherein R is 8-18 carbon atoms.

5. A surfactant composition according to any one of the preceding claims wherein the triglyceride is coconut oil, palm oil, sunflower oil, soybean oil, rapeseed oil, castor oil, fish oil, tallow fat, milk fat, and/or lard.

6. A surfactant composition according to claim 5 wherein the triglyceride is coconut oil and/or soybean oil.

7. A method for preparing a modified monoethanolamide surfactant composition having a congealing temperature of 20°C or lower which comprises
(a) providing a monoethanolamide composition, prepared from triglyceride, represented by the formula (2) 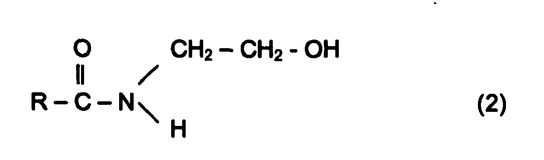 wherein
R is optionally substituted or unsubsitututed, branched or straight chain, saturated or unsaturated, hydrocarbon radical of 3-21 carbon atoms; and
b) reacting 1 mole of said monoethanolamide composition in the presence of a suitable catalyst with 1-6 moles of propylene oxide, butylene oxide or mixtures of the same, to form a modified monoethanolamide surfactant composition that is substantially liquid at 25°C or lower.

8. A method according to claim 7 wherein R is 8-18 carbon atoms.

9. A method according to either one of claims 7 and 8 wherein the monoethanolamide composition is reacted with from 1 to 4 moles of propylene oxide.

10. A method according to any one of claims 7 to 9 wherein the monoethanolamide composition to be reacted is a solid having a congealing temeperature of at least 40°C.

11. A method according to any one of claims 7 to 10 wherein the monoethanolamide composition is prepared from coconut oil, palm oil, sunflower oil, soybean oil, rapeseed oil, castor oil, fish oil, tallow fat, milk fat, and/or lard triglycerides.

12. A method according to claim 11 wherein the monoethanolamide composition is prepared from coconut oil and/or soybean oil.

13. Cosmetic, personal care and household use compositions which comprise at least 0.1% by weight of a modified monoethanolamide surfactant composition as defined in any one of claims 1 to 6.

14. The use of a monoethanolamide composition prepared from glyceride esters, to form a modified monoethanolamide surfactant composition of the formula (1) 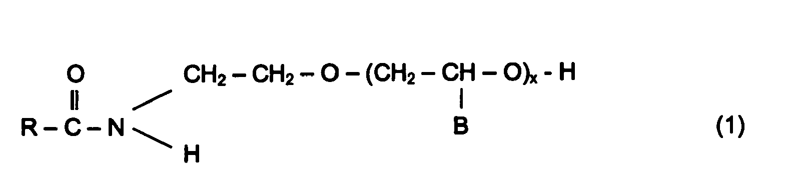 wherein
R is optionally substituted or unsubstituted, branched or straight chain, saturated or unsaturated or unsaturated hydrocarbon radical of 3-21 carbon atoms,
B is CH₃ or -CH₂ - CH₃, and
x is from 1 to 6;
having a congealing temperature of 20°C or lower, and being substantially liquid at 25°C or lower.

15. The use according to claim 14 wherein the monoethanolamide composition is prepared from coconut oil, palm oil, sunflower oil, soybean oil, rapeseed oil, castor oil, fish oil, tallow fat, milk fat, and/or lard.

16. The use according to either one of claims 14 and 15 wherein the modified monoethanolamide surfactant composition exhibits foam stabilization and viscosity building properties.

17. Metal working and industrial use compositions which are in liquid form comprising at least 0.1% by weight of a modified monoethanolamide surfactant composition as defined in any one of claims 1 to 6.

## Patentansprüche

1. Tensidzusammensetzung eines modifizierten Monoethanolamids, abgeleitet von aus Triglycerid hergestelltem Monoethanolamid, mit einer Erstarrungstemperatur von 20°C oder niedriger, von der Formel (1) 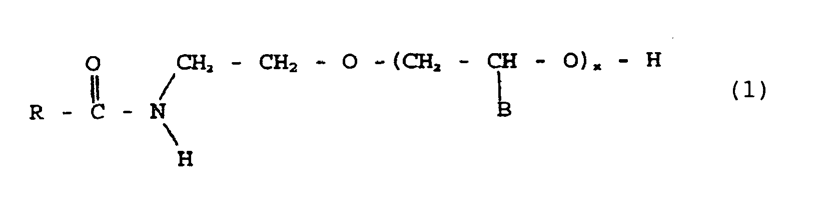 wobei
R ein wahlweise substituiertes oder unsubstituiertes, verzweigtes oder geradkettiges, gesättigtes oder ungesättigtes Kohlenwasserstoffradikal mit 3-21 Kohlenstoffatomen ist,
B ist CH₃ oder -CH₂-CH₃, und
X ist von 1 bis 6;
die Zusammensetzung eines modifizierten Monoethanolamids im Wesentlichen flüssig bei 25°C oder niedriger ist, und geeignet ist, Schaumstabilisierung und viskositätsbildende Eigenschaften aufzuweisen.

2. Tensidzusammensetzung nach Anspruch 1, wobei x von 1 bis 4 ist.

3. Tensidzusammensetzung nach einem der Ansprüche 1 oder 2, wobei B eine CH₃-Gruppe ist.

4. Tensidzusammensetzung nach einem der vorangehenden Ansprüche, wobei R hat 8-18 Kohlenstoffatome.

5. Tensidzusammensetzung nach einem der vorangehenden Ansprüche, wobei das Triglycerid ist Kokosnussöl, Palmöl, Sonnenblumenöl, Sojaöl, Rapsöl, Castoröl, Tran, Talgfett, Milchfett und/oder Schmalz.

6. Tensidzusammensetzung nach Anspruch 5, wobei das Triglycerid Kokosnussöl und/oder Sojaöl ist.

7. Verfahren zum Herstellen einer Tensidzusammensetzung eines modifizierten Monoethanolamids mit einer Erstarrungstemperatur von 20°C oder weniger, das umfasst
(a) Bereitstellen einer Monoethanolamid-Zusammensetzung, hergestellt aus Triglycerid, dargestellt durch die Formel (2) 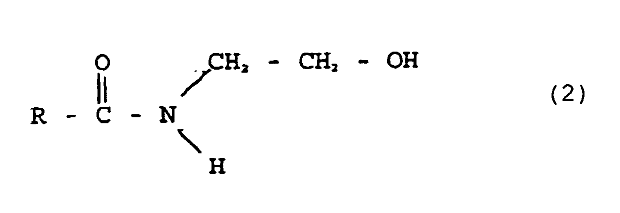 wobei
R ein wahlweise substituiertes oder unsubstituiertes, verzweigtes oder geradkettiges, gesättigtes oder ungesättigtes Kohlenwasserstoffradikal mit 3-21 Kohlenstoffatomen ist; und
(b) Reagieren von 1 Mol der Monoethanolamid-Zusammensetzung in der Anwesenheit eines geeigneten Katalysators mit 1-6 Mol Propylenoxid, Butylenoxid oder Gemischen desselben, um eine Tensidzusammensetzung eines modifizierten Monoethanolamids zu bilden, die im Wesentlichen flüssig bei 25°C oder weniger ist.

8. Verfahren nach Anspruch 7, wobei R hat 8-18 Kohlenstoffatome.

9. Verfahren nach einem der Ansprüche 7 oder 8, wobei die Monoethanolamid-Zusammensetzung reagiert wird mit 1 bis 4 Mol Propylenoxid.

10. Verfahren nach einem der Ansprüche 7 bis 9, wobei die zu reagierende Monoethanolamid-Zusammensetzung ein Feststoff mit einer Erstarrungstemperatur von mindestens 40°C ist.

11. Verfahren nach einem der Ansprüche 7 bis 10, wobei die Monoethanolamid-Zusammensetzung hergestellt wird aus Kokosnussöl, Palmöl, Sonnenblumenöl, Sojaöl, Rapsöl, Castoröl, Tran, Talgfett, Milchfett und/oder Schmalz Triglyceriden.

12. Verfahren nach Anspruch 11, wobei die Monoethanolamid-Zusammensetzung hergestellt wird aus Kokosnussöl und/oder Sojaöl.

13. Kosmetik-, Körperpflege- und Haushaltsgebrauchs-Zusammensetzungen, die umfassen mindestens 0,1 Gew-% einer Tensidzusammensetzung eines modifizierten Monoethanolamids, wie in einem der Ansprüche 1 bis 6 definiert.

14. Verwendung einer Monoethanolamid-Zusammensetzung, hergestellt aus Glyceridestern, um eine Tensidzusammensetzung eines modifizierten Monoethanolamids der Formel (1) zu bilden 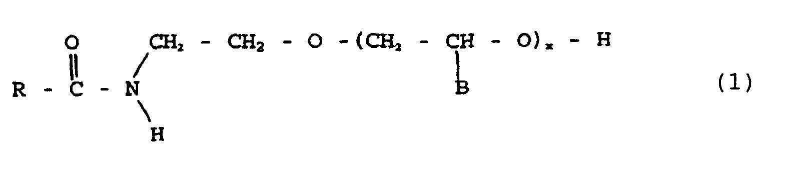 wobei
R ein wahlweise substituiertes oder unsubstituiertes, verzweigtes oder geradkettiges, gesättigtes oder ungesättigtes Kohlenwasserstoffradikal mit 3-21 Kohlenstoffatomen ist,
B ist CH₃ oder -CH₂-CH₃, und
x ist von 1 bis 6;
mit einer Erstarrungstemperatur von 20°C oder niedriger, und im Wesentlichen flüssig bei 25°C oder niedriger.

15. Verwendung nach Anspruch 14, wobei die Monoethanolamid-Zusammensetzung hergestellt wird aus Kokosnussöl, Palmöl, Sonnenblumenöl, Sojaöl, Rapsöl, Castoröl, Tran, Talgfett, Milchfett und/oder Schmalz.

16. Verwendung nach einem der Ansprüche 14 oder 15, wobei die Tensidzusammensetzung des modifizierten Monoethanolamids aufweist Schaumstabilisierung und viskositätsbildende Eigenschaften.

17. Metallverarbeitungs- und Industrieanwendungszusammensetzungen, die in flüssiger Form sind, umfassend mindestens 0,1 Gew-% einer Tensidzusammensetzung eines modifizierten Monoethanolamids, wie definiert in einem der Ansprüche 1 bis 6.

## Revendications

1. Composition tensioactive de mono-éthanolamide modifié dérivée de mono-éthanolamide préparé à partir de triglycérides, présentant une température de gélification de 20° C ou inférieure, de formule (1) 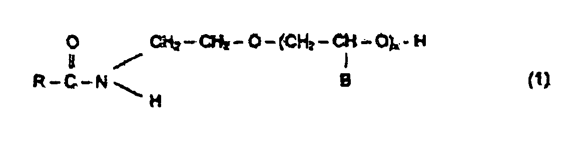 dans laquelle
R est un radical hydrocarbure éventuellement substitué ou non substitué, à chaîne linéaire ou ramifiée, saturé ou insaturé comportant 3 à 21 atomes de carbone,
B est CH₃ ou -CH₂-CH₃, et
x vaut de 1 à 6 ;
ladite composition de mono-éthanolamide modifié étant essentiellement liquide à 25° C ou à une température inférieure et étant appropriée pour présenter des propriétés de stabilisation de mousse et de formation de viscosité.

2. Composition tensioactive selon la revendication 1, dans laquelle x vaut de 1 à 4.

3. Composition tensioactive selon l'une quelconque des revendications 1 et 2, dans laquelle B est un groupe CH₃.

4. Composition tensioactive selon l'une quelconque des revendications précédentes, dans laquelle R désigne 8 à 18 atomes de carbone.

5. Composition tensioactive selon l'une quelconque des revendications précédentes, dans laquelle le triglycéride est l'huile de noix de coco, l'huile de palme, l'huile de tournesol, l'huile de soja, l'huile de colza, l'huile de ricin, l'huile de poisson, de la graisse de suif, de la graisse de lait et/ou de saindoux.

6. Composition tensioactive selon la revendication 5, dans laquelle le triglycéride est l'huile de noix de coco et/ou l'huile de soja.

7. Procédé de préparation d'une composition tensioactive de mono-éthanolamide modifié présentant une température de gélification de 20° C ou inférieure, comprenant
(a) la fourniture d'une composition de mono-éthanolamide préparée à partir de triglycérides, représentée par la formule (2) 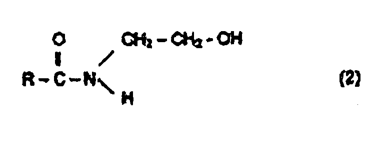 dans laquelle
R est un radical hydrocarbure éventuellement substitué ou non substitué, à chaîne linéaire ou ramifiée, saturé ou insaturé comportant 3 à 21 atomes de carbone,
et
b) la réaction d'une mole de ladite composition de mono-éthanolamide en présence d'un catalyseur approprié avec 1 à 6 moles d'oxyde de propylène, d'oxyde de butylène ou de mélanges de ceux-ci, pour former une compositior tensioactive de mono-éthanolamide modifié qui est essentiellement liquide à 25° C ou à une température inférieure.

8. Procédé selon la revendication 7, dans lequel R désigne 8 à 18 atomes de carbone.

9. Procédé selon l'une quelconque des revendications 7 et 8, dans lequel la composition de mono-éthanolamide réagit avec 1 à 4 moles d'oxyde de propylène.

10. Procédé selon l'une quelconque des revendications 7 à 9, dans lequel la composition de mono-éthanolamide à faire réagir est un solide présentant une température de gélification d'au moins 40° C.

11. Procédé selon l'une quelconque des revendications 7 à 10, dans lequel la composition de mono-éthanolamide est préparée à partir de triglycérides d'huile de noix de coco, d'huile de palme, d'huile de tournesol, d'huile de soja, d'huile de colza, d'huile de ricin, d'huile de poisson, de la graisse de suif, de la graisse de lait et/ou de saindoux.

12. Procédé selon la revendication 11, dans lequel la composition de mono-éthanolamide est préparée à partir d'huile de noix de coco et/ou d'huile de soja.

13. Compositions utilisées pour les cosmétiques, le soin personnel et l'entretien domestique qui comprennent au moins 0,1 % en poids d'une composition tensioactive de mono-éthanolamide modifié telle que définie dans l'une quelconque des revendications 1 à 6.

14. Utilisation d'une composition de mono-éthanolamide préparée à partir d'esters de glycérides, pour former une composition tensioactive de mono-éthanolamide modifié de formule (1) 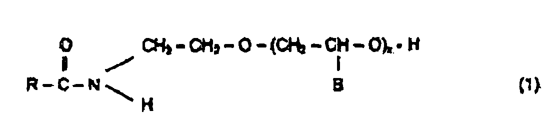 dans laquelle
R est un radical hydrocarbure éventuellement substitué ou non substitué, à chaîne linéaire ou ramifiée, saturé ou insaturé comportant 3 à 21 atomes de carbone,
B est CH₃ ou -CH₂-CH₃, et
x vaut de 1 à 6 ;
présentant une température de gélification de 20° C ou inférieure et étant essentiellement liquide à 25° C ou à une température inférieure.

15. Utilisation selon la revendication 14, dans laquelle la composition de mono-éthanolamide est préparée à partir d'huile de noix de coco, d'huile de palme, d'huile de tournesol, d'huile de soja, d'huile de colza, d'huile de ricin, d'huile de poisson, de la graisse de suif, de la graisse de lait et/ou de saindoux.

16. Utilisation selon l'une quelconque des revendications 14 et 15, dans laquelle la composition tensioactive de mono-éthanolamide présente des propriétés de stabilisation de mousse et de formation de viscosité.

17. Compositions utilisées dans le travail des métaux et dans les applications industrielles, qui sont de forme liquide, comprenant au moins 0,1 % en poids d'une composition tensioactive de mono-éthanolamide modifié telle que définie dans l'une quelconque des revendications 1 à 6.
